# EUROPEAN PATENT APPLICATION

(11) **EP 4 202 468 A1**
(43) Date of publication of application: **28.06.2023**
(21) Application number: 21217639.0
(22) Date of filing: 23.12.2021
(51) Int. Cl.: G01R 33/56, A61B 5/055, G01R 33/561, G06T 7/00, G06V 10/764, G06N 3/02

(54) **DIRECT INFERENCE BASED ON UNDERSAMPLED MRI DATA OF HUMANS OR ANIMALS**

(71) Applicant: Orbem GmbH, 85748 Garching b.Munich (DE)
(72) Inventor: Coello Uribe, Jorge Eduardo, 80802 München (DE); Kudielka, Guido, 71384 Weinstadt (DE); Gómez Damián, Pedro Agustín, 80802 München (DE); Laparidou, Maria, 80634 München (DE); Molina Romero, Miguel, 80939 München (DE)
(74) Representative: Pelster Behrends Patentanwälte PartG mbB

(57) **Abstract**

The present invention relates to a computer implemented method for identification of at least one predetermined medically relevant feature in undersampled MRI data (300) of a living being of a predefined species with an inference module (200), the method comprising the steps of: a) analysing undersampled MRI data (300) of a living being of the predefined species for identifying at least one predetermined medically relevant feature using a machine learning module (204) that is trained for identifying the at least one predetermined medically relevant feature in living beings of the predefined species from undersampled MRI data (300), and b) classifying the undersampled MRI data (300) of the living being of the predefined species based on the result of the identification of the at least one predetermined medically relevant feature, wherein the undersampled MRI data (300) comprises undersampled raw MRI data, comprising a plurality of time dependent signals for different phases, and/or processed MRI data, obtained from processing undersampled raw MRI data, wherein the method is conducted using an inference module (200), comprising a memory (202) storing the machine learning module (204) and a processor (206) for controlling the inference module (200), wherein the inference module is configured to provide the undersampled MRI data (300) as an input to the machine learning module (204), to analyse the undersampled MRI data (300) using the machine learning module (204) and to classify the undersampled MRI data (300), and wherein the machine learning module (204) is trained for identifying the at least one predetermined medically relevant feature in living beings of the predefined species from undersampled MRI data (300) using a training set (302) comprising undersampled MRI data (300) of different training samples of the predefined species, wherein for each predetermined medically relevant feature a fraction of the training samples comprises the predetermined medically relevant feature and a fraction of the training samples does not comprise the predetermined medically relevant feature.

## Description

The present invention relates to a computer implemented method for automated identification of at least one predetermined medically relevant feature in MRI data of a living being of a predefined species with an inference module, an inference module using a machine learning module, preferably in a respective method, an MRI system comprising said inference module, and a computer program product to carry out the steps of the respective method.

In order to improve the chances of successfully treating diseases and other medical conditions in humans or animals, powerful diagnostic methods are required that allow for an early, precise and reliable identification of medical conditions in order to choose the correct treatment. Therefore, the improvement of diagnostic methods and the development of enhanced devices for use in such methods is an important focus in the medical field. Well-known and powerful tools for use in diagnostic methods are NMR (nuclear magnetic resonance) based techniques and in particular Magnetic Resonance Imaging (MRI). The use of MRI techniques allows for the examining of living beings for providing information about their interior in a non-invasive diagnostic method. Typically, the resulting MRI images are then analysed by medical experts that identify any significant deviation from the standard, sometimes called symptom, that is a medically relevant feature of the respective living being. Using the medical experts' knowledge, the medically relevant feature would be attributed to derive a diagnosis about the underlying medical condition.

Recording MRI images of living beings that are of sufficient quality for the analysis by a medical expert oftentimes require significant measurement time. This is typically considered an unfavorable feature of MRI measurements. Patients, in particular children, often consider it unpleasant to remain in the device for the entire time required for the measurement, in particular as typical MRI scanners feature comparably small measurement zones that can make the measurement even more unpleasant for the patient. From a socioeconomic perspective, the main disadvantage is that long measurement times reduce the available capacities of a healthcare facility, increasing the demand for equipment and/or reducing the availability of MRI based analysis for the population.

An approach for saving measurement time and for increasing the processing rate is to reduce the digitization rate and/or the number of experiments taken in the phase encoding dimension. This results in the acquisition of undersampled MRI data. Furthermore, repetition times for each scan and/or the number of scans taken in each sub-experiment can be reduced to further increase the processing rate. Using this approach, the overall time required for each patient can be reduced, in particular when combined with techniques like parallel imaging.

However, the acquisition of undersampled MRI data in the so-called k-space, results in MRI images that have a reduced quality and in particular exhibit so-called aliasing artefacts, while shorter repetition times and lower number of scans can drastically reduce the signal-to-noise ratio and the overall quality of the obtainable MRI image. Yet, prior art MRI-based diagnostic methods are mostly based around the visual inspection of the MRI images by a medical expert, thereby requiring a functional minimum of image quality in order to allow a reliable diagnosis. Herein it can be detrimental, if e.g. the location of a lesion in the human body is misjudged due to a fold-back aliasing artefact.

As a consequence, the prior art is heavily focussed on improving the quality of MRI images obtained from undersampled MRI data using different approaches, including some concepts that rely on artificial intelligence and utilize e.g. artificial neural networks for removing artefacts from images. Related disclosure can e.g. be found in US 2021/224634 A1, US 11,170,543 B2 and US 2020/0305756 A1.

While these techniques for improving the image quality are in several cases effective at obtaining high quality MRI images from undersampled MRI data, the respective reconstruction of aliased MRI images based on undersampled MRI data typically requires powerful processors and large memory capacities, i.e. high performance computers. The amount of additional computational power required for enhanced image reconstruction adds to the initial resources needed for performing the complex multi-dimensional Fourier transform that is required for obtaining the basic MRI image from the undersampled MRI data in the first place. Depending on the available computational power, even the overall image reconstruction itself can become a factor that reduces the overall measurement speed.

The combination of long measurement times with a high demand for computational power makes MRI techniques less attractive for fast screening experiments, in that a large number of patients need to be quickly checked for specific features or in that a preventive screening is employed to a large number of individuals, e.g. even outside the hospital. This is further amplified by the fact that the dependence on visual inspection of the MRI images by medical experts limits the rate of analysis, making e.g. the retrospective review of larger amounts of old MRI data that is e.g. stored in a database, very difficult. Furthermore, such MRI techniques are often difficult to employ in areas with an insufficient digital infrastructure and/or after catastrophic events that may cause a surge of patients that are in need for treatment, due to the reduced availability of computational resources or limited band-width for providing MRI data to a server in order to provide for a centralized image processing.

It was the primary objective of the present invention to overcome or at least reduce the disadvantages of the prior art.

In particular, it was the objective of the present invention to provide for way of enabling MRI based diagnosis with shorter experiment times and reduced demand for computational resources, thereby increasing MRI capacities of medical facilities.

It was an objective of the present invention, to provide for a way of increasing the usability of MRI based diagnosis methods for fast screening of several patients, in particular in order to identify patients that should be examined more carefully by medical experts.

Herein, it was an objective of the present invention, to provide for a way for increasing the degree of automation in MRI based diagnostic methods.

It was a further objective of the present invention, to provide for a way for efficiently analysing large amounts of archived MRI data that was e.g. recorded for other purposes, in order to identify medically relevant features that afterwards become of interest and to allow identification of patients that should be examined more carefully by medical experts.

It was an objective of the present invention, to provide for a way to make MRI based diagnosis methods more accessible in areas with insufficient infrastructure capacity, in particular areas that were affected by a catastrophic event.

It was a further objective of the present invention, to provide for a way of facilitating a decentralized processing of MRI data and/or to support remote diagnostics by medical experts, in particular in areas with an insufficient infrastructure capacity.

It was desired that the respective solution to the above objectives should be applicable to a wide variety of living beings, i.e. usable in the human and veterinary medicine, and a broad range of medically relevant features, wherein it should be able to obtain very reliable results and should be compatible with a wide variety of typical MRI scanners.

The inventors of the present invention have now found, that the above described objectives can surprisingly be achieved with a computer implemented method for automated identification of at least one predetermined medically relevant feature in undersampled MRI data of a living being of a predefined species, that uses a specific inference module as defined in the claims. Herein, the inventors realized that the above objectives can be achieved when MRI and machine learning techniques are combined to facilitate the analysis of undersampled MRI data. In particular, the inventors found that by utilizing machine learning an efficient screening for a specific medically relevant feature and even a corresponding initial prediction of the probability of a corresponding medical condition does not require that MRI images are provided as an output and/or that a visual inspection is made by a medical expert. Instead it is in several cases sufficient to automatically identify a predetermined medically relevant, i.e. its presence, absence or magnitude, wherein it is sufficient to provide the respective information as an output, rather than a processed MRI image. Insofar, the inventors developed an approach based on machine learning that synergistically utilizes this idea. For this, the inventors did not rely on techniques of increasing the MRI image quality but instead found that machine learning techniques can be directly applied to undersampled MRI data, in particular undersampled raw MRI data and MRI images that comprise aliasing artefacts, in order to identify, if a predefined medically relevant feature is present in the living being or not. In fact, it was found that aliasing artefacts, that are known to result from processing undersampled MRI raw data and in several case lead to a replication of features, like e.g. of a lesion, can surprisingly be utilized for synergistically enhancing the identification capability of said feature by a machine learning module that is trained for this purpose. Thus, the inventors found that surprisingly advantage of the aliasing artefacts could be taken instead of trying to mitigate it, as the inventors observed that aliasing artefacts that result from processing of undersampled raw MRI data can be engineered to replicate features of interest. During development, the inventors found that this beneficial concept can be utilized even further, as the machine learning module, unlike humans, is not dependent on analysing fully processed MRI images. Instead, it was found, that the method can surprisingly be conducted to identify predetermined medically relevant features directly from undersampled raw MRI data or processed MRI data that is not fully transformed into an MRI image, e.g. obtained after a two or three dimensional Fourier transform, thereby beneficially reducing the amount of computational resources needed for the general image reconstruction. In other words, the inventors found that the strategy to limit the search to one or more specific medically relevant predefined features of interest in combination with a powerful machine learning technique and an adequate training, enables a direct inference based on undersampled MRI data, thus allowing for very high analysis rate and a very reliable analysis of the presence of medically relevant features. Advantageously, the computer implemented method can be applied to a broad variety of living beings and medically relevant features and is compatible with undersampled MRI data obtained by all typical MRI scanners. Furthermore, the computer implemented method allows for an increased level of automation in MRI based diagnostics.

Thanks to the above described strategy, it is e.g. possible to use MRI techniques for efficient screening. For example, several short MRI measurements can be used to collect undersampled MRI data from a large number of patients, wherein the computer implemented method is used to quickly identify those patients that show a medically relevant feature and therefore should be examined in more detail, wherein this analysis is possible despite the low data quality and without the need for sophisticated image improvement. Likewise, the computer implemented method can efficiently be used to review archived undersampled MRI data that requires low memory space, in order to identify a medically relevant feature of interest in old undersampled MRI data that was originally taken for a different purpose. This is of high interest if a new feature is identified to be of potential medical relevance. If for example a certain change to the vascular system in the legs, that was originally considered unproblematic, is later found to be connected to increased risk of suffering a specific medical condition, the computer implemented method may be used to efficiently analyse the archived undersampled MRI data of patients that were diagnosed with MRI due to e.g. a torn ligament in the leg, in order to identify patients that should be examined in more detail with respect to the specific medical condition.

The above described objectives are therefore solved by the subject-matter of the present invention as defined in the claims. Hereinafter, the subject-matter of the invention is discussed in more detail, wherein preferred embodiments of the invention are disclosed. It is particularly preferred to combine two or more of the preferred embodiments to obtain an especially preferred embodiment. Correspondingly, especially preferred is a method according to the invention, that defines two or more features of preferred embodiments of the present invention. Also preferred are embodiments in which a feature of one embodiment that is to some extent designated as preferred is combined with one or more further features of other embodiments that are designated to some extent as preferred. Features of preferred MRI Systems, inference modules and computer program products result from features of preferred methods.

The present invention relates to a computer implemented method for identification of at least one predetermined medically relevant feature in undersampled MRI data of a living being of a predefined species with an inference module, the method comprising the steps of:
a) analysing undersampled MRI data of a living being of the predefined species for identifying at least one predetermined medically relevant feature using a machine learning module that is trained for identifying the at least one predetermined medically relevant feature in living beings of the predefined species from undersampled MRI data, and
b) classifying the undersampled MRI data of the living being of the predefined species based on the result of the identification of the at least one predetermined medically relevant feature,
wherein the undersampled MRI data comprises undersampled raw MRI data, comprising a plurality of time dependent signals for different phases, and/or processed MRI data, obtained from processing undersampled raw MRI data, wherein the method is conducted using an inference module, comprising a memory storing the machine learning module and a processor for controlling the inference module, wherein the inference module is configured to provide the undersampled MRI data as an input to the machine learning module, to analyse the undersampled MRI data using the machine learning module and to classify the undersampled MRI data, and wherein the machine learning module is trained for identifying the at least one predetermined medically relevant feature in living beings of the predefined species from undersampled MRI data using a training set comprising undersampled MRI data of different training samples of the predefined species, wherein for each predetermined medically relevant feature a fraction of the training samples comprises the predetermined medically relevant feature and a fraction of the training samples does not comprise the predetermined medically relevant feature.

The method of the present invention comprises the identification of a predetermined medically relevant feature in a plurality of living beings of a predefined species.

The term "living being" as used herein refers to living creatures, in particular humans and animals. In agreement with the understanding of the skilled person, the term "MRI data of a living being" denotes the origin of the MRI data, i.e. that it was acquired in an MRI measurement on the respective living being. The physical presence of said living being when performing the computer implemented method is neither necessary nor desired, in particular in the case of animals.

The living being who's undersampled MRI data is analysed in the computer implemented method according to the invention belong to a predefined species. The term "species" as used herein relates to the basic unit of classification used in biology to classify living beings. The species is a "predefined species" indicating that the computer implemented method of the invention is configured for a specific species that is defined prior to conducting the computer implemented method of the present invention. The computer implemented method of the invention can be limited to subspecies or other smaller grouping criterions for living beings in a species. In other words, the term "predefined species" is a grouping criterion to group a number of biologically similar living beings. While this definition might appear unwieldy at first, there is no practical obstacle for the skilled person to define a species and to decide, if a living being falls under the predefined species. For example, the predefined species may be "homo sapiens" or "gallus gallus". Insofar, the skilled person readily understands that a limitation to a subspecies or even smaller grouping criterions, like e.g. living beings of a species with similar sex or age, typically will enhance the accuracy of the computer implemented method of the present invention and can reduce the requirements for the training set for training the machine learning module. Thus, it is preferred for most cases to define the living beings as narrow as reasonably possible using appropriate grouping criterions and to adapt the training set accordingly.

The living beings of the predefined species can in principle be any living being from any species. However, the inventors have found that the method of the present invention can be employed to certain living beings in a very efficient way. Specifically, a computer implemented method is preferred, wherein the predefined species is selected from the group consisting of humans, birds, fish and domestic animals, preferably humans, birds, pets and livestock, more preferably, humans, dogs, cats, chicken, cattle and horses, most preferably humans. Herein, the use of the computer implemented method for analysis of undersampled MRI data of humans is preferred for all embodiments.

The term "predetermined medically relevant feature" as used herein refers to a medically relevant feature that has been specified, set or established in advance of the method of the present invention, wherein "predetermined" and "predefined" are herein used interchangeably and attributed to the "medically relevant feature" and the "species", respectively, to allow for better distinguishing between the two aspects. In principle the predetermined medically relevant feature can be any characteristic, property, or attribute of the living organism. Insofar, the term "medically relevant" merely serves as a descriptor to clearly define the use of the computer implemented method and to distinguish the features that are to be identified in the computer implemented method from other uses of the word "feature". However, the skilled person understands that the computer implemented method of the invention can be applied to generic "predefined features" as well. In practice this difference is of no relevance, as basically all features that are derivable from undersampled MRI data of living beings should be medically relevant. Herein, a computer implemented method is preferred, wherein the predetermined medically relevant feature is selected from the group consisting of physiological features, anatomical features and pathological features, preferably lesions, more preferably ruptures, infections, tumors, tissue abnormalities, organ abnormalities and changes to the vascular system.

Compared to most prior art techniques, that analyse quality enhanced MRI images visually and derive conclusions from what is visible (either visually or with regular computer programs), the method of the present invention searches for a specific predefined medically relevant feature in the undersampled MRI data, by training the machine learning module for this respective medically relevant feature.

The skilled person understands that the method according to the invention can be used to simultaneously identify two or more predefined medically relevant features on the same undersampled MRI data, e.g. the presence of a fracture and a change in the vascular system.

The undersampled MRI data that is analysed in the computer implemented method can be provided in several ways, e.g. from an archive or other database of MRI data. Likewise, the inference module could act as a server for receiving and analysing the undersampled MRI data that is provided by clients, e.g. hospitals or MRI facilities.

In all scenarios undersampled MRI data can be recorded on living beings using the typical experiments and typical MRI scanners. The general structure and function of MRI scanners and the concepts of MRI measurements, including typical MRI experiments, pulse sequences and techniques are well known to the skilled person and need not be described herein. MRI scanners are commercially available from several suppliers and in most cases include the software and pulse programs required for recording MRI data or undersampled MRI data, respectively. More disclosure about the general concept of MRI is readily available in the prior art, e.g. from WO 2019/092265 A1. With respect to the type of undersampled MRI data, the computer implemented method can be adapted to basically all types of undersampled MRI data, irrespective of the type of experiment, measurement parameters, nuclei or other parameters, wherein only the training set for training the machine learning module should be adapted to sufficiently correspond to the format and/or type of the undersampled MRI data

For understanding the method of the present invention, understanding the distinction between "undersampled MRI data", "undersampled raw MRI data" and "processed MRI data obtained from processing undersampled raw MRI data" is of relevance as well as the difference to regular, non-undersampled MRI data. In agreement with the skilled persons understanding, MRI data comprises the raw MRI data that is obtained in an MRI measurement by sampling (or digitizing) the continuous time dependent NMR signal (e.g. FID or echo) for a set of different experiments in the two- or three-dimensional experiment (also referred to a k-space) as well as any representations of undersampled raw MRI data that are derived thereof by processing, in particular by Fourier transforming or applying any form of linear or nonlinear encoding to the data, preferably by Fourier transforming. To obtain "undersampled MRI data" in an MRI experiment, the sampling is performed on less points than intrinsically necessary to resolve the highest frequency contributing to the continuous time dependent NMR signal and/or to resolve the phase angle of the magnetization, respectively. For example, for the time dimension, the sampling rate for obtaining the discrete time dependent signal is below twice the highest frequency, i.e. the Nyquist rate, of the continuous time dependent NMR signal. A corresponding concept applies to the number of signals for different phases, that can also be described as time dependent signals with different spatial frequency components, wherein undersampling happens if the spacing between the phase increments is larger than the corresponding Nyquist interval. In agreement with the skilled persons understanding, the term "undersampled raw MRI data" comprises all minor modifications of undersampled raw MRI data that are merely arbitrary modifications like addition or subtraction of a fixed value or multiplication with a fixed value.

Apart from the undersampled raw MRI data, the term "undersampled MRI data" also comprises processed MRI data obtained from processing undersampled raw MRI data, wherein the processing can comprise in principle any linear on nonlinear data processing operation but in most cases will comprise a Fourier transform in one, two or three dimensions, eventually leading to an MRI image that comprises aliasing artefacts that are caused by the undersampling. Both the Nyquist rate and the Nyquist interval are well known to the skilled person and recording of undersampled MRI data that can be used in the computer implemented method according to the invention poses no problem for the skilled person.

Undersampling allows for particularly fast MRI measurements and generally enables a high measurement rate. This advantageous effect is even more pronounced, when the MRI data is undersampled in two dimensions or even three dimensions in case not only one slice is recorded and a three dimensional image shall be constructed using two phase-encoding dimensions. While undersampling in one dimension is typically considered "bad enough" in the prior art, undersampling in two or more dimensions is oftentimes considered not expedient in methods of the prior art. However, it is a large advantage of the present invention that the computer implemented method according to the invention can readily be employed for reliable feature identification using MRI data that is undersampled in two or more dimensions and/or wherein the undersampling is particular severe by sampling with a large difference to the Nyquist rate and the Nyquist interval. Therefore, the computer implemented method according to the invention enables an efficient way of identification of medically relevant features in MRI data despite the high degree of undersampling, thereby allowing for faster MRI measurements and an increase of diagnostic capacity. As heavily undersampled MRI data also requires less memory capacity, undersampled MRI data can be stored more easily and can be transmitted much faster even at low bandwidth. Thus, using heavily undersampled MRI data is very advantageous in areas with an insufficient infrastructure capacity, e.g. areas that were affected by a catastrophic event.

In view of the above explanations, a computer implemented method according to the invention is preferred, wherein the undersampled MRI data was obtained in an MRI measurement, wherein the sampling rate for the time dependent signal is below the Nyquist rate and/or the spacing between the phase increments is larger than the Nyquist interval, wherein preferably the sampling rate for the time dependent signal is set to be below the Nyquist rate and the spacing between the phase increments is set to be above the Nyquist interval.

In view of the above explanations, a computer implemented method according to the invention is preferred, wherein the undersampled MRI data was obtained in an MRI measurement, wherein the sampling rate for the time dependent signal is 75 % or less, preferably 50 % or less, more preferably 25 % or less, of the Nyquist rate, and/or wherein the spacing between the phase increments is 200 % or more, preferably 300 % or more, more preferably 400 % or more, of the Nyquist interval. This corresponds to skipping lines in the k-space. Most preferably, the respective features are realized together with the corresponding degrees of preference.

Generally, the undersampling or the reduction of the sampling rate, respectively, can be achieved along any encoding dimension, that is along frequency, phase, or slice encoding.

As discussed above, undersampled MRI data may comprise either or both of "undersampled raw MRI data" and "processed MRI data obtained from processing undersampled raw MRI data". The latter refers to any type of processed MRI data obtained from the undersampled raw MRI data, particularly by performing a Fourier transformation in the phase-encoding and/or the time-encoding dimension, preferably in both dimensions. The skilled person understands that it is expedient that no artificial generation of additional sampling points or any other method of increasing the data quality is performed on the processed MRI data, as such steps are not needed by the method of the invention.

Due to being based on undersampled raw MRI data, the processed MRI data obtained therefrom by Fourier transform along an undersampled dimension comprises at least one aliasing artefact. Within the skilled persons understanding, the aliasing artefact is an effect that causes different signals of the continuous-time NMR signal to become indistinguishable in the discrete time dependent signal. While these aliasing artefacts are often detrimental for prior art methods, they advantageously do not hinder the method according to the invention, at least not too much, and can even be beneficial for training the machine learning module as aliasing artefacts can result in duplication of the predetermined medically relevant feature in the sample, providing more features for the machine learning module to recognize. Correspondingly, if undersampled raw MRI data is obtained, a method according to the invention can be preferred, wherein the analysing comprises processing undersampled raw MRI data by Fourier transforming along at least one undersampled dimension, to obtain processed MRI data that comprises at least one aliasing artefact.

The highest processing rates as well as the strongest reduction in required computational power is typically accessible if undersampled raw MRI data is directly analysed, wherein the exclusive analysis of undersampled raw MRI data is a preferred option in all embodiments. Overall, the capability of the machine learning module to identify the predetermined medically relevant features in living beings in undersampled raw MRI data, in particular in undersampled k-space data, and the option of removing any Fourier transform or image processing steps, is among the most relevant advantages of the computer implemented method according to the invention. Particularly preferred is therefore a computer implemented method according to the invention, wherein the undersampled MRI data, comprises undersampled raw MRI data, wherein the undersampled MRI data, preferably consists of undersampled raw MRI data. As indicated above, preferred is also a computer implemented method according to the invention, wherein the undersampled raw MRI data is undersampled in both the time dimension and the phase dimension. Correspondingly, a method according to the invention is preferred, wherein the machine learning module is trained for identifying the predetermined medically relevant feature in living beings of the predefined species from undersampled raw MRI data.

It was found that training of machine learning modules for identifying the predetermined medically relevant feature in living beings of the predefined species from undersampled raw MRI data can be more challenging than providing more processed data, thus e.g. requiring larger training sets. For several applications it is therefore preferred to use processed MRI data. Correspondingly, a computer implemented method according to the invention is preferred, wherein the undersampled MRI data, comprises processed MRI data, wherein the undersampled MRI data, preferably consists of processed MRI data.

Herein, a very preferred route uses highly processed MRI data and even MRI images with aliasing artefacts, in particular as several MRI scanners are optimized for returning MRI images as an output so that the respective data is sometimes more readily available. In view of this, a computer implemented method according to the invention is preferred, wherein the processed MRI data is obtained from processing undersampled raw MRI data using a one-, two- or three-dimensional, preferably two-dimensional, Fourier Transform, in particular along the frequency-encoding dimension and/or the phase-encoding dimension, and/or wherein the processed MRI data is the one-, two- or three-dimensional, preferably two-dimensional, Fourier transform of undersampled raw MRI data, in particular along the frequency-encoding dimension and/or the phase-encoding dimension.

Likewise preferred is a computer implemented method according to the invention, wherein the processed MRI data is obtained from processing undersampled raw MRI data by Fourier transforming along at least one undersampled dimension, to obtain processed MRI data that comprises at least one aliasing artefact, wherein the machine learning module is trained for identifying the predetermined medically relevant feature in living beings of the predefined species from processed MRI data that comprises at least one aliasing artefact. Particularly preferred is a computer implemented method according to the invention, wherein the processed MRI data is an MRI image obtained by Fourier transforming or applying any form of linear or nonlinear encoding to undersampled MRI raw data, preferably by Fourier transforming, undersampled MRI raw data along an undersampled frequency-encoding dimension and/or an undersampled phase-encoding dimension, wherein the MRI image comprises at least one aliasing artefact, wherein the machine learning module is trained for identifying the predetermined medically relevant feature in living beings of the predefined species from MRI images that comprise at least one aliasing artefact.

However, a very efficient computer implemented method according to the invention is obtained as a good compromise between efficiency of the analysis and required computational power if a computer implemented method according to the invention is employed, wherein the processed MRI data is obtained by processing undersampled raw MRI data by Fourier transforming along exactly one undersampled dimension, to obtain processed MRI data that comprises at least one aliasing artefact. The use of undersampled raw MRI data and/or processed MRI data with a low degree of processing rather than full MRI images was found to be a very advantageous embodiment.

The computer implemented method of the present invention comprises the step of analysing the undersampled MRI data with an inference module for identifying the predetermined medically relevant feature in the living being. The skilled person understands that the result of this step is a feedback of the inference module and thus the information if the predetermined medically relevant feature is present in the living being or present in a specific magnitude, respectively. Therefore, the step a) of the computer implemented method according to the invention for example returns a comparably simple answer in the form of "yes", "no" or "partially", thus contributing to the advantageous fast processing rates. In view of the underlying concept of the computer implemented method according to invention it is neither necessary nor expedient that the inference module also returns MRI data, in particular processed MRI data, as this would in most cases only increase the amount of required computational power and the need for additional hardware, e.g. in the form of displays. Therefore, a computer implemented method according to the invention is preferred, wherein the inference module does not comprise means for outputting visualized output of processed MRI data, in particular MRI images. Likewise, a computer implemented method according to the invention is preferred, wherein the inference module is not configured to improve the resolution of an MRI image and/or to reconstruct an MRI image and/or to remove artefacts from an MRI image and/or to improve the quality of an MRI image in another way, wherein most preferably none of these process steps are performed in the method according to the invention. Especially preferred is a method according to the invention, wherein no processed MRI data is provided as an output of the inference module.

The inference module used in the computer implemented method of the present invention is used for analysing the undersampled MRI data for identifying a predetermined medically relevant feature of the living being. The inference module itself is a physical device and can be a typical data processing device, e.g. a computer. In agreement with this, the inference module comprises a memory, i.e. a computer-readable storage, for storing e.g. data or software as well as a processor, i.e. a digital circuit which is able to perform operations on an external data source, for controlling the inference module. As the hardware infrastructure of the inference module is not critical for the method of the present invention, it is expedient to define the presence of only the basic components of an electronic data processing system, i.e. the memory and the processor, wherein for both of these components typical elements can be used that are commercially available, wherein the inference module can also comprise other components of typical data processing systems, e.g. power supply, mouse, keyboard, displays, network connectors etc.

The processor of the inference module is used for controlling the inference module, wherein the inference module is configured to provide the undersampled MRI data as an input to the machine learning module and to analyse the undersampled MRI data using the machine learning module. The skilled person understands that this configuration is established in the typical way, e.g. by using software that can be programmed by the skilled person himself or provided by typical specialized programming companies. Thus, the inference module typically comprises computer executable code, that can be stored in the memory, which comprise machine executable instructions or a program which causes a processor and the inference module to perform their respective tasks in the method of the present invention. Suitable designs for assemblies from processors and memory are e.g. disclosed in EP 3 704 666 B1.

The core component of the inference module is the machine learning module that is stored in the inference module's memory, and therefore usable by the inference module for analysing the undersampled MRI data by providing the undersampled MRI data to the machine learning module. The machine learning module processes the input and identifies the predetermined medically relevant feature, in particular the presence, magnitude or absence of the predetermined medically relevant feature, wherein the output of the machine learning module is processed by the inference module and the processor, respectively, wherein the output of the machine learning module can e.g. be stored in a memory, provided to a user via an interface or provided as an input into subsequent processing devices, e.g. sorters.

In recent years, employing machine learning techniques or so-called artificial intelligence has become a promising approach for solving different technical problems in several industrial fields. Today, machine learning, its core concepts and its implementation are well-known concepts and several companies offer commercial solutions for implementing machine learning. While some skilled persons that have their focus in the field of MRI and/or in the field of quality control would potentially have to study some literature before setting up some of the more complex machine learning solutions entirely on their own, this skill is not necessarily required for putting the method of the invention into practice. Suitable machine learning algorithms and codes are available from the prior art and from commercial service providers if required. In practice, in most industries the implementation of the machine learning aspects of the present invention will probably be provided by skilled persons in the field of machine learning that have no problems in providing a suitable machine learning module in view of the disclosure of the invention, wherein these skilled persons can e.g. be part of a team of employees or personal of external IT-service providers. Insofar, the machine learning module is similar to the MRI scanner in that it oftentimes will be provided by specialized companies.

The inference module uses the machine learning module for analysing the undersampled MRI data. In agreement with the understanding of the skilled person, the machine learning module is the entity, e.g. a program, that is actually analysing the undersampled MRI data and can provide for the desired identification of the predetermined medically relevant feature, typically without task-specific programming. For this, the machine learning module typically comprises both data and a procedure for using the data to make a prediction, sometimes called prediction algorithm. The machine learning module itself does not necessarily need to be able to further "learn" or "train" itself, but can exist, e.g. in the form of a program comprising the machine learning module, and continuously serve its task in the same way.

In agreement with the skilled persons understanding, machine learning modules can be obtained by running (or fitting) a machine learning algorithm, sometimes called learning rule or learning algorithm, on a data set, that is oftentimes called training set. Thus, by running a machine learning algorithm on a training set of data a machine learning module can be obtained or an existing machine learning module can be modified. Thus, by using the machine learning algorithm on the training set the machine learning module is trained for its specific task. In the framework of the present invention and in agreement with the established wording, the machine learning module is defined to be trained, wherein the training is specified in particular by defining the training set. An exemplary disclosure for the use of machine learning in the field of MRI can be found in EP 3 704 666 B1, that provides additional information for further understanding the technological background.

In practice, the choice of the machine learning algorithm used for obtaining the machine learning module will in most cases depend on the living beings to be analysed and, oftentimes more importantly, the predetermined medically relevant feature that needs to be identified. Yet, the inventors were able to identify suitable machine learning algorithms that can be used to generate the machine learning module, that is correspondingly based on the respective machine learning algorithm. In particular, a method according to the invention is preferred, wherein the machine learning module is based on a machine learning algorithm, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms. Correspondingly, a method according to the invention is preferred, wherein the machine learning module is obtained by applying a machine learning algorithm on the training set, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms. Herein, the machine learning algorithms themselves are known in the prior art and can be employed by the skilled person in view of the living being and the predetermined medically relevant feature.

Based on this, the inventors identified specific types of machine learning modules that have proven to provide excellent results in the identification of predetermined medically relevant features in living beings and that are therefore explicitly preferred for all embodiments. Therefore, a method according to the invention is especially preferred, wherein the machine learning module is a deep learning network or an artificial neural network, preferably a deep learning network. Herein, these types of machine learning modules are known to the skilled person and e.g. described in more detail in EP 3 704 666 B1.

Due to the large amounts of undersampled MRI data that are typically processed using the computer implemented method of the present invention, it is highly recommended to provide for a mechanism for continued training of the machine learning module, to continuously enhance the performance and accuracy of the identification. Thus, a method according to the invention is especially preferred, wherein the inference module comprises a machine learning algorithm for generating a machine learning module or training a machine learning module based on a set of undersampled raw MRI data collected by using the method, wherein the machine learning algorithm is selected from the group consisting of regression algorithms, linear classifiers, instance-based algorithms, regularization algorithms, decision tree algorithms, bayesian algorithms, clustering algorithms, association rule learning algorithms, artificial neural network algorithms, deep learning algorithms, dimensionality reduction algorithms and ensemble algorithms, preferably linear classifiers, artificial neural network algorithms and deep learning algorithms.

While the skilled person chooses the machine learning algorithm based on his general knowledge and his specific needs, it is the training of the machine learning module and therefore the training set that connects the machine learning modules of the present invention with each other. According to the invention, the machine learning module is trained for identifying the at least one predetermined medically relevant feature in living beings of the predefined species using a training set comprising undersampled MRI data recorded for different training samples of the predefined species, wherein a fraction of the training samples comprises the predetermined medically relevant feature and a fraction of the training samples does not comprise the predetermined medically relevant feature. Therefore, the training of the machine learning module is a supervised training, i.e. a training based on input data with known results. In this training process predictions are made, which are manually or automatically corrected when turning out wrong. The desired level of accuracy is achieved through repetitions of the aforementioned process.

The training samples for the training of the machine learning module for identifying the predetermined medically relevant feature in living beings of the predefined species are therefore represented by living beings of the same predefined species, i.e. training samples of undersampled MRI data in dogs for identifying predetermined medically relevant features in undersampled MRI data of dogs, with a known result, i.e. whether the dogs exhibit the predetermined medically relevant feature or not. In other words, the presence or absence of the predetermined medically relevant feature in the training samples is known prior to the training of the machine learning module. It is known that the accuracy of the prediction typically i.a. depends on the quantity and quality of the training samples. For some applications, it might be beneficial to include training samples that are represented by living beings of a species that is similar to the predefined species, e.g. to increase the amount of available training data if the deviations between the species are not very relevant for the planed analysis.

For some machine learning based techniques, e.g. for improvement of image quality or for the removal of artefacts like disclosed in e.g. EP 3 704 666 B1, methods are suggested for generating an artificial training set. However, the inventors found that this is only possible in the present case, if the predetermined medically relevant feature is sufficiently represented in the artificial training set. Given the variability of size, location, and morphology of these features, this can be very difficult to achieve. On the other hand, due to the fact that the method according to the invention is typically employed on large amounts of undersampled MRI data, e.g. from archived undersampled MRI data, potential undersampled MRI data of real living beings that exhibit the predetermined medically relevant feature (or not, respectively) are typically available in sufficient numbers. Herein, the use of training sets that are obtained on real living beings with real predetermined medically relevant features is preferred due to typically yielding the most potent machine learning modules.

The inventors found that the best training results for the machine learning module are typically obtained, if the amount of training samples that comprises the predetermined medically relevant feature is comparably large. Specifically, a computer implemented method according to the invention is preferred, wherein the fraction of the training samples that comprises the predetermined medically relevant feature is in the range of 20 to 80 %, preferably in the range of 30 to 70 %, more preferably in the range of 40 to 60 %, most preferably in the range of 45 to 55 %. Advantageously, if two or more predetermined medically relevant features shall be identified, the machine learning module can in most cases be trained with a training set in that the amount of training samples that do not exhibit any of the predetermined medically relevant features can be significantly reduced, as training samples that only exhibit feature A can be used as a negative example for feature B. In particular, a computer implemented method is preferred, wherein a fraction of the training samples comprises at least a first predetermined medically relevant feature and a fraction of the training samples comprises at least a second predetermined medically relevant feature, wherein preferably the fraction of training samples that comprises at least one of the first or second predetermined medically relevant feature is the range of 40 to 100 %, preferably in the range of 60 to 95 %, more preferably in the range of 70 to 90 %.

Generally, a computer implemented method is preferred, wherein the machine learning module is trained for identifying at least one predetermined medically relevant feature in living beings of the predefined species using the training set, wherein each undersampled MRI data of different training samples is linked with information about the MRI scanner and/or the experimental parameters of the MRI measurement used to obtain the undersampled MRI data, so that the machine learning module is trained for identifying the predetermined medically relevant feature in living beings of the predefined species in undersampled MRI data obtained with a plurality of different MRI scanners and/or under different experimental conditions, wherein the inference module is configured to provide information about the MRI scanner and/or the experimental parameters used for generating the analysed undersampled MRI data as an input to the machine learning module, wherein the experimental parameters are preferably selected from the group comprising pulse lengths, pulse sequence, evolution times, repetitions times, sampling rate, phase increments, temperature and number of scans, or wherein the training set comprises undersampled MRI data of different training samples of the predefined species that was recorded with the same type of MRI scanner used for generating the analysed undersampled MRI data, wherein preferably similar, more preferably basically identical, experimental parameters were employed for recording the undersampled MRI data of the training samples as are used for generating the analysed undersampled MRI data.

The skilled person is aware that the above definition of the computer implemented method according to the invention and the training of the machine learning module implies that the training set comprises undersampled MRI data in the same format as the analysed undersampled MRI data. The skilled person that intends to analyse undersampled k-space MRI data would not consider a machine learning module that was trained on undersampled 3D-MRI-lmages.For the same reason, the skilled person is aware that a computer implemented method is preferred, wherein the training set comprises undersampled MRI data of the same body portion of the living beingof the predefined species as the analysed undersampled MRI data.

In the computer implemented method of the invention, the undersampled MRI data of the living being of the predefined species, and therefore indirectly the living beings, is classified based on the result of the identification of at least one predetermined medically relevant feature. This means that two or more classes are provided that are based on a grouping criterion, wherein the living beings and their undersampled MRI data, respectively, are assigned to one or more of these classes based on the identification of the predetermined medically relevant feature. For example, if the predetermined medically relevant feature is the rupture of a ligament, the classes could e.g. be i) no rupture, ii) minor rupture and iii) severe rupture, wherein e.g. living beings of class iii) would be selected for a more detailed examination first. Insofar, a computer implemented method is preferred, wherein the classification is based on the presence, absence or magnitude of at least one predetermined medically relevant feature, and/or wherein the classification is made in 5 or more, preferably 10 or more, more preferably 20 or more, classes.

Taking into account the above disclosure, it is understood that a large benefit of the present invention consists of the high usability and reliability of the fast identification of predetermined medically relevant features and the fast classification of the living beings that makes the computer implemented method of the present invention particularly suited to decide about further treatment and/or diagnostics. Thus, a computer implemented method is preferred, wherein a further treatment and/or diagnostic procedures of the living being is conducted in dependence of the presence, absence or magnitude of the predefined medically relevant feature. Alternatively, or additionally, a computer implemented method is preferred, wherein a further analysis of the undersampled MRI data is conducted in dependence of the presence, absence or magnitude of the predefined medically relevant feature.

In a preferred embodiment, the above classification is used to automatically make a prediction about the existence of at least one medical condition in the living being. Therefore, a computer implemented method is preferred, further comprising the step of:
c) predicting the probability of the existence of at least one medical condition in the living being of the predefined species,
wherein the inference module is configured to predicting the probability of the existence of at least one medical condition based on the classification, preferably by referring to a list stored in the memory that attributes probabilities of the existence of the at least one medical condition to the possible classifications, and/or wherein the machine learning module is trained for predicting the probability of the existence of at least one medical condition in the living being of the predefined species from the undersampled MRI data, wherein the training set comprises information about the presence, absence or magnitude of the at least one medical condition in the living beings used for generating the training samples.

Overall, a computer implemented method is preferred, wherein the inference module is configured to provide the classification as an output.

One of the main advantages of the computer implemented method of the invention lies in the potential of quickly analysing large amounts of undersampled MRI data with a comparably low demand for computational power, wherein the size of the data packages can be reduced, in particular when using undersampled raw MRI data. In view of this, it is preferred to utilize this advantage by applying the method to large amounts of undersampled MRI data and/or to provide for a decentralised data analysis. Therefore, a computer implemented method is preferred, wherein the method is operated at a rate of 10 10 or more analysed undersampled MRI data of different living beings per hour, preferably 20 or more per hour, more preferably 100 or more per hour, most preferably 1000 or more per hour. Likewise, a computer implemented method is preferred, wherein the method is applied sequentially or in parallel, preferably in parallel, for a collection of undersampled MRI data comprising undersampled MRI data from 2 or more, preferably 10 or more, more preferably 20 or more, most preferably 50 or more, living beings of the predefined species. Finally, a computer implemented method is preferred, wherein the interference module is a decentralized data processing device, wherein the undersampled MRI data is preferably provided to the interference module by a plurality of client data processing devices that are connected to MRI scanners.

It is clear to the skilled person, that the invention also relates to an inference module for automated identification of at least one predetermined medically relevant feature in undersampled MRI data of a living being of a predefined species, using a machine learning module, preferably in a computer implemented method according to the invention,
wherein the inference module comprises a memory storing the machine learning module and a processor for controlling the inference module,
wherein the inference module is configured to provide the undersampled MRI data as an input to the machine learning module, to analyse the undersampled MRI data using the machine learning module and to classify the undersampled MRI data,
wherein the machine learning module is trained for identifying the at least one predetermined medically relevant feature in living beings of the predefined species from undersampled MRI data using a training set comprising undersampled MRI data of different training samples of the predefined species, wherein for each predetermined medically relevant feature a fraction of the training samples comprises the predetermined medically relevant feature and a fraction of the training samples does not comprise the predetermined medically relevant feature.

In view of this, the skilled person understands that the present invention likewise relates to an MRI system comprising:
a) an MRI scanner for obtaining undersampled MRI data of living beings of a predefined species, and
b) an inference module according to the invention, that is connected to the MRI scanner.

The invention also relates to a computer program product comprising instructions which, when the program is executed by a computer, preferably by an inference module according to the invention, cause the computer to carry out the step a), preferably the steps a) and b), more preferably all steps, of the computer implemented method according to the invention.

Also disclosed herein are a computer-readable data carrier having stored thereon the computer program product according to the invention as well as a data carrier signal carrying the computer program product according to the invention.

Hereinafter, the invention is described in more detail, wherein preferred embodiments of the invention are disclosed with respect to the figures. The figures show:
- Fig. 1: a schematic visualization of the steps of the computer implemented method according to the invention;
- Fig. 2: a schematic visualization of the structure of an inference module according to the invention; and
- Fig. 3: a schematic visualization of the principle of the computer implemented method according to the invention.

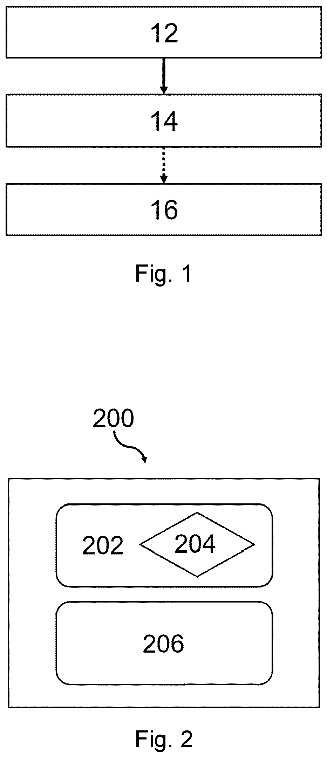

Fig. 1 provides a schematic visualization of an exemplary computer implemented method according to the invention for identification of at least one predetermined medically relevant feature in undersampled MRI data of a living being of a predefined species to facilitate the understanding of the present invention.

In a first step 12 of the computer implemented method, undersampled MRI data 300 of a living being of the predefined species is analysed with an inference module 200. The inference module 200 comprises a memory 202 with a machine learning module 204 stored thereon and a processor 206, which is configured to control the inference module 200. The analysing procedure within the inference module 200 in the first step 12 is performed using the machine learning module 204, wherein the undersampled MRI data 300 serves as an input to the inference module 200 and the machine learning module 204, respectively.

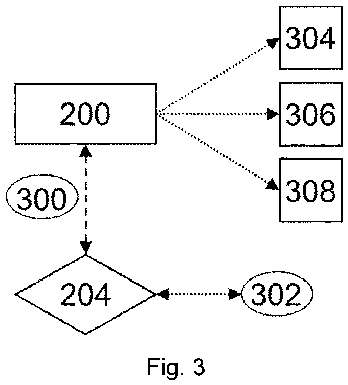

A schematic visualization of the structure of an inference module 200 that is suitable for use in the computer implemented method according to the invention, in particular for the computer implemented method shown in fig. 1 and 3, is provided in fig. 2. Herein, the machine learning module 204 is trained for identifying the at least one predetermined medically relevant feature in living beings of the predefined species.

The training is made using a suitable machine learning algorithm and a training set 302 comprising undersampled MRI data 300 of different training samples of living beings of the predefined species in order to allow for supervised training. The training set 302 for example consists of undersampled MRI data 300 of training samples of living beings of the predefined species with and without the predetermined medically relevant feature. In other words, a fraction of the training samples comprises the predetermined medically relevant feature and a fraction of the training samples does not comprise the predetermined feature. For example, the fraction of the training samples in the training set 302 that comprises the predetermined medically relevant feature is in the range of 45 to 55 %. Based on this training of the machine learning module 204, the computer implemented method is able to identify the presence, absence or magnitude of the predetermined medically relevant feature.

In the preferred embodiment depicted in fig. 2, the inference module 200 does not comprise means for providing visualized output of processed MRI data like MRI images, for example in the form of a display. Correspondingly, the inference module 200 is not configured to improve the quality of MRI images in any way but rather is configured to provide the result of the identification and potentially of the classification, for example to subsequent processing devices like e.g. sorting devices.

In an especially preferred embodiment of the inference module 200 according to fig. 2, that is a preferred option for all embodiments of the computer implemented method, the machine learning module 204 is based on an artificial neural network algorithm or deep learning algorithm. Accordingly, the machine learning module is an artificial neural network or a deep learning module, wherein preferably the inference module 200 also comprises the machine learning algorithm for further training the machine learning module based on the undersampled MRI data 300 analysed in the computer implemented method of the invention.

Fig. 1 also depicts the second step 14. In this second step 14, the undersampled MRI data 300 is classified by the inference module 200. The classification is based on the result of the analysis performed in the first step 12, wherein the result comprises information on the presence and/or absence and/or magnitude of the predetermined medically relevant feature in the living beings. For example, the undersampled MRI data 300 of the living being can be classified in three classes 304, 306 and 308, as is schematically visualized for an exemplary computer implemented method according to the invention in fig. 3. In this example, the first class 304, the second class 306 and the third class 308 correspond to the classification as "not comprising the medically relevant feature", "comprising the medically relevant feature", and "unclear/repeat", wherein those living beings who's undersampled MRI data 300 was classified in the second class are prioritized when deciding about which living being needs to be examined more carefully by a medical expert. Finally, any undersampled MRI data 300 that was classified as "unclear" in the third class 308, can e.g. be analysed again and/or reviewed by a medical expert.

In a preferred embodiment, the classification performed in the second step 14 is used for predicting the probability of the existence of at least one medical condition in the living being of the predefined species in a third step 16.

### Reference Signs

- 12: process step a)
- 14: process step b)
- 16: process step c)

- 200: inference module
- 202: memory
- 204: machine learning module
- 206: processor

- 300: undersampled MRI data
- 302: training set
- 304: first class
- 306: second class
- 308: third class

## Claims

1. Computer implemented method for identification of at least one predetermined medically relevant feature in undersampled MRI data (300) of a living being of a predefined species with an inference module (200), the method comprising the steps of:
a) analysing undersampled MRI data (300) of a living being of the predefined species for identifying at least one predetermined medically relevant feature using a machine learning module (204) that is trained for identifying the at least one predetermined medically relevant feature in living beings of the predefined species from undersampled MRI data (300), and
b) classifying the undersampled MRI data (300) of the living being of the predefined species based on the result of the identification of the at least one predetermined medically relevant feature,
wherein the undersampled MRI data (300) comprises undersampled raw MRI data, comprising a plurality of time dependent signals for different phases, and/or processed MRI data, obtained from processing undersampled raw MRI data, wherein the method is conducted using an inference module (200), comprising a memory (202) storing the machine learning module (204) and a processor (206) for controlling the inference module (200), wherein the inference module is configured to provide the undersampled MRI data (300) as an input to the machine learning module (204), to analyse the undersampled MRI data (300) using the machine learning module (204) and to classify the undersampled MRI data (300), and wherein the machine learning module (204) is trained for identifying the at least one predetermined medically relevant feature in living beings of the predefined species from undersampled MRI data (300) using a training set (302) comprising undersampled MRI data (300) of different training samples of the predefined species, wherein for each predetermined medically relevant feature a fraction of the training samples comprises the predetermined medically relevant feature and a fraction of the training samples does not comprise the predetermined medically relevant feature.

2. Computer implemented method according to claim 1, further comprising the step of:
c) predicting the probability of the existence of at least one medical condition in the living being of the predefined species,
wherein the inference module (200) is configured for predicting the probability of the existence of at least one medical condition based on the classification, preferably by referring to a list stored in the memory (202) that attributes probabilities of the existence of the at least one medical condition to the possible classifications, and/or wherein the machine learning module (204) is trained for predicting the probability of the existence of at least one medical condition in the living being of the predefined species from the undersampled MRI data (300), wherein the training set (302) comprises information about the presence, absence or magnitude of the at least one medical condition in the living beings used for generating the training samples.

3. Computer implemented method according to any one of claims 1 or 2, wherein the undersampled MRI data (300), comprises undersampled raw MRI data, wherein the undersampled MRI data (300), preferably consists of undersampled raw MRI data.

4. Computer implemented method according to any one of claims 1 to 3, wherein the undersampled MRI data (300), comprises processed MRI data, wherein the undersampled MRI data (300), preferably consists of processed MRI data.

5. Computer implemented method according to any one of claims 1 to 4, wherein the processed MRI data is obtained as an MRI image by Fourier transforming or applying any form of linear or nonlinear encoding to the undersampled MRI raw data along an undersampled frequency-encoding dimension or an undersampled phase-encoding dimension, wherein the MRI image comprises at least one aliasing artefact, wherein the machine learning module (204) is trained for identifying the at least one predetermined medically relevant feature in living beings of the predefined species from undersampled MRI data (300) that comprises at least one aliasing artefact, wherein the classification is based on the presence, absence or magnitude of at least one predetermined medically relevant feature.

6. Computer implemented method according to any one of claims 1 to 5, wherein the inference module (200) is configured to provide the classification as an output.

7. Computer implemented method according to any one of claims 1 to 6, wherein the interference module (200) is a decentralized data processing device, wherein the undersampled MRI data (300) is preferably provided to the interference module by a plurality of client data processing devices that are connected to MRI scanners.

8. Computer implemented method according to any one of claims 1 to 7, wherein the machine learning module (204) is trained for identifying at least one predetermined medically relevant feature in living beings of the predefined species using the training set (302), wherein each undersampled MRI data (300) of different training samples is linked with information about the MRI scanner (106) and/or the experimental parameters of the MRI measurement used to obtain the undersampled MRI data (300), so that the machine learning module (204) is trained for identifying the predetermined medically relevant feature in living beings of the predefined species in undersampled MRI data (300) obtained with a plurality of different MRI scanners (106) and/or under different experimental conditions, wherein the inference module (200) is configured to provide information about the MRI scanner (106) and/or the experimental parameters used for generating the analysed undersampled MRI data (300) as an input to the machine learning module (204), wherein the experimental parameters are preferably selected from the group comprising pulse lengths, pulse sequence, evolution times, repetitions times, sampling rate, phase increments, temperature and number of scans.

9. Computer implemented method according to any one of claims 1 to 8, wherein a fraction of the training samples comprises at least a first predetermined medically relevant feature and a fraction of the training samples comprises at least a second predetermined medically relevant feature, wherein preferably the fraction of training samples that comprises at least one of the first or second predetermined medically relevant feature is the range of 40 to 100 %, preferably in the range of 60 to 95 %, more preferably in the range of 70 to 90 %.

10. Computer implemented method according to any one of claims 1 to 9, wherein the machine learning module (204) is a deep learning network or an artificial neural network, preferably a deep learning network.

11. Computer implemented method according to any one of claims 1 to 10, wherein the predefined species is selected from the group consisting of humans, birds, fish and domestic animals, preferably humans, birds, pets and livestock, more preferably, humans, dogs, cats, chicken, cattle and horses, most preferably humans.

12. Computer implemented method according to any one of claims 1 to 11, wherein the predetermined medically relevant feature is selected from the group consisting of physiological features, anatomical features and pathological features, preferably lesions, more preferably fractures, infections, tumors, tissue abnormalities, organ abnormalities and changes to the vascular system.

13. Inference module (200) for automated identification of at least one predetermined medically relevant feature in undersampled MRI data (300) of a living being of a predefined species, using a machine learning module (204), preferably in a computer implemented method according to any one of claims 1 to 12,
wherein the inference module (200) comprises a memory (202) storing the machine learning module (204) and a processor (206) for controlling the inference module (200),
wherein the inference module (200) is configured to provide the undersampled MRI data (300) as an input to the machine learning module (204), to analyse the undersampled MRI data (300) using the machine learning module (204) and to classify the undersampled MRI data (300),
wherein the machine learning module (204) is trained for identifying the at least one predetermined medically relevant feature in living beings of the predefined species from undersampled MRI data (300) using a training set (302) comprising undersampled MRI data (300) of different training samples of the predefined species, wherein for each predetermined medically relevant feature a fraction of the training samples comprises the predetermined medically relevant feature and a fraction of the training samples does not comprise the predetermined medically relevant feature.

14. MRI system, comprising:
a) an MRI scanner for obtaining undersampled MRI data (300) of living beings of a predefined species, and
b) an inference module (200) according to claim 13, that is connected to the MRI scanner.

15. Computer program product comprising instructions which, when the program is executed by a computer, preferably by an inference module (200) according to claim 13, cause the computer to carry out the steps of the computer implemented method according to any one of claims 1 to 12.
